# EUROPÄISCHE PATENTSCHRIFT

(11) **EP 1 118 613 B1**
(45) Veröffentlichungstag und Bekanntmachung des Hinweises auf die Patenterteilung: **24.09.2003**
(21) Anmeldenummer: 00126142.9
(22) Anmeldetag: 30.11.2000
(51) Int. Cl.: C07D 301/12

(54) **Verfahren zur Herstellung von Olefinoxiden in der Gasphase**
Process for the production of olefinic oxides in the gas phase
Procédé pour la preparation d'oxides olefinique dans la phase du gas

(30) Priorität: 21.01.2000 DE 10002514
(43) Veröffentlichungstag der Anmeldung: 25.07.2001
(73) Patentinhaber: Degussa AG, 40474 Düsseldorf (DE); Uhde GmbH, 44141 Dortmund (DE)
(72) Erfinder: Schütte, Rüdiger, Dr., 63755 Alzenau (DE); Markowz, Georg, Dr., 63791 Karlstein (DE); Esser, Peter, Dr., 45657 Recklinghausen (DE); Balduf, Torsten, Dr., 63456 Hanau (DE); Thiele, Georg, Dr., 63450 Hanau (DE); Hasenzahl, Steffen, Dr., 63477 Maintal (DE)

(56) Entgegenhaltungen:
- US-A- 4 374 260
- T.M. NAGIYEV ET AL: "Gas-phase Oxidation of Propylene by hydrogen peroxide" PETROL. CHEM. , Bd. 31, Nr. 5, 1991, Seiten 670-677, XP002168795
- T HAYASHI ET AL: "JOURNAL OF CATALYSIS,US,ACADEMIC PRESS, DULUTH, MN" JOURNAL OF CATALYSIS,US,ACADEMIC PRESS, DULUTH, MN, Bd. 178, Nr. 2, 1998, Seiten 566-575, XP002114496 ISSN: 0021-9517

## Beschreibung

Die Erfindung richtet sich auf ein Verfahren zur Herstellung von Olefinoxiden mit 2 bis 6 C-Atomen, insbesondere Propenoxid, durch Gasphasenepoxidation des entsprechenden Olefins mit 2 bis 6 C-Atomen mit Wasserstoffperoxid in Gegenwart eines festen Katalysators.

Die Epoxidation von Olefinen, wie Propen, unter Einsatz von Wasserstoffperoxid gelingt in der Flüssigphase unter Einsatz eines Titansilikalitkatalysators - siehe US 5,874,596 und DE 197 31 627. Nachteilig an diesem Verfahren ist die rasche Deaktivierung des Katalysators durch hochsiedende Nebenprodukte.

Bekannt ist auch die Flüssigphasen-Epoxidation von Olefinen mit Wasserstoffperoxid in Gegenwart eines molybdän- oder wolframhaltigen Katalysators - siehe Weigert et al., Chem.-Ztg. 99, 19 (1975). Die Aufarbeitung des Reaktionsgemischs und Rückgewinnung des Katalysators sind hierbei recht aufwendig.

Aus der CA 2,206,626 A1 ist auch bekannt, die Epoxidation in einem Membranreaktor durchzuführen, wobei in der Composit-Membran katalytisch wirksame Partikel eingelagert sind und sich auf der einen Seite der Membran eine Gasphase mit dem zu epoxidierenden Olefin, wie Propen, und auf der anderen Seite eine flüssige Phase mit Wasserstoffperoxid befinden. Die katalytisch wirksamen Partikel bestehen vorzugsweise aus Titansilikalit.

Anstelle in flüssiger Phase lässt sich Ethylen auch in der Gasphase in Gegenwart eines silberhaltigen Katalysators bei 200 bis 300 °C epoxidieren, als Epoxidierungsmittel wird hierbei aber nicht Wasserstoffperoxid, sondern Luft oder molekularer Sauerstoff verwendet (siehe beispielsweise US-Patent 4,374,260).

Es wurde auch versucht, niedere Olefine mit Wasserstoffperoxid in der Gasphase zu epoxidieren, wobei Wasserstoffperoxid thermisch oder katalytisch aktiviert wird: So lassen sich gemäß G.M. Mamedjarov und T.M. Nagiev (Azerb. Khim. Zh. (1981), 57-60) Ethen und Propen bei 500 bis 600 °C in der Gasphase in Abwesenheit eines Katalysators epoxidieren. Die zunächst niedrigen Ausbeuten an Epoxid konnten von T.M. Nagiev et al. (Neftekhimiya 31 (1991), 670-675) durch Optimierung auf etwa 50 bis 55 % Propenoxid gesteigert werden. Nachteilig sind die hohen Reaktionstemperaturen, die einem wirtschaftlichen Prozess entgegenstehen.

Den Mechanismus eines ähnlichen Verfahrens untersuchten H.M. Gusenov et al. (Azerb. Khim. Zh. (1984), 47-51), jedoch erfolgt die Gasphasen-Epoxidation hier in Gegenwart eines Si-haltigen Katalysators bei 425 bis 500 °C. Propen und dampfförmiges Wasserstoffperoxid werden einem Rohrreaktor zugeführt; der Propenumsatz liegt im Bereich von 15 bis 65 %.

Eine verbesserte Gasphasenepoxidation in Gegenwart eines Fe-haltigen Katalysators lehren T.M. Nagiev et al. (Neftekhimiya 31 (1991), 670-675): Unter Einsatz von Magnetit als Katalysator wird Propen mit Wasserstoffperoxid bei etwa 250 °C mit einer Ausbeute um 30 % zu Propenoxid epoxidiert. Die Katalysatorstandzeit ist mit 25 h aber sehr gering. Eine höhere Standzeit und weitere Absenkung der Reaktionstemperatur kann mit einem an Aluminiumoxid als Träger gebundenen Fe^{III}OH-Protoporphyrin-Katalysator erhalten werden. Mit diesem Katalysator wird bei einer Temperatur um 160 °C und einem Einsatzmolverhältnis von C₃H₆:H₂O₂:H₂O = 1:0,2:0,8 eine Propenoxidausbeute von etwa 50 % erhalten.

Aufgabe der Erfindung ist es, ein weiteres Verfahren zur katalytischen Gasphasenepoxidation niederer Olefine mit Wasserstoffperoxid aufzuzeigen, wobei der Katalysator eisenfrei sein und die Epoxidation unterhalb 250 °C durchführbar sein sollte. Der Begriff "eisenfrei" schließt hier die Anwesenheit von Eisenspuren im Katalysator im Umfang üblicher Verunreinigungen nicht aus.

Die Aufgabe wird gelöst durch ein Verfahren zur Herstellung eines Olefinoxids mit 2 bis 6 C-Atomen, insbesondere Propylenoxid, durch Gasphasenepoxidation des zu epoxidierenden Olefins mit Wasserstoffperoxid, umfassend Kontaktieren eines das Olefin, Wasserstoffperoxid und Wasser enthaltenden gasförmigen Gemischs mit einem festen Katalysator und Isolieren des Olefinoxids aus dem Reaktionsgemisch, das dadurch gekennzeichnet ist, dass man als Katalysator eine Verbindung eines Elements der 4. bis 6. Nebengruppe des Periodensystems der Elemente oder von Arsen oder Selen oder ein Molekularsieb verwendet und die Epoxidation bei einer Temperatur unterhalb 250 °C in Abwesenheit einer flüssigen Phase durchführt.

Die Unteransprüche richten sich auf bevorzugte Ausführungsformen des erfindungsgemäßen Verfahrens.

Bei einer Klasse geeigneter Katalysatoren handelt es sich um Molekularsiebe, insbesondere synthetische Zeolithe. Ein besonders bevorzugter Katalysator aus der Reihe der Molekularsiebe basiert auf titanhaltigen Molekularsieben der allgemeinen Formel (SiO₂)₁₋ₓ(TiO₂)ₓ, wie Titansilikalit-1 (TS-1) mit MFI-Kristallstruktur, Titansilikalit-2 (TS-2) mit MEL-Kristallstruktur, Titan-Beta-Zeolith mit BEA-Kristallstruktur und Titansilikalit-48 mit der Kristallstruktur von Zeolith ZSM 48. Der TiO₂-Gehalt in TS-1 liegt vorzugsweise im Bereich von 2 bis 4 %. Titansilikalite sind im Handel erhältlich. Anstelle reiner Titansilikalite können auch Kombinationsprodukte, welcher außer Titansilikalit auch amorphe oder kristalline Oxide, wie SiO₂, TiO₂, Al₂O₃, ZrO₂ enthalten. Hierbei können Kristallite von Titansilikalit mit den Kristalliten der anderen Oxide homogen verteilt sein und Granulate bilden oder sich als äußere Schale auf einem Kern aus anderen Oxiden befinden.

Bei einer weiteren Klasse erfindungsgemäß zu verwendender Katalysatoren handelt es sich vorzugsweise um anorganische, insbesondere oxidische Verbindungen, welche als katalysatorwirksames Element ein oder mehrere Elemente der 4. bis 6. Nebengruppe des Periodensystems oder eine Arsen- oder Selenverbindung enthalten. Bevorzugt handelt es sich um Verbindungen von Titan, Zirkon, Vanadium, Niob, Tantal, Chrom, Molybdän und Wolfram. Die katalytische Wirkung wird, ohne andere Mechanismen auszuschließen, darin gesehen, dass Wasserstoffperoxid durch die poröse Struktur des Katalysators und/oder durch die Befähigung des Katalysators zur reversiblen Ausbildung von Peroxoverbindungen aktiviert wird.

Beispiele geeigneter Katalysatoren sind Vanadiumoxide, Vandate, Niob- und Tantaloxid und -oxidhydrate sowie H₂O₂-Addukte der genannten Oxide und Oxidhydrate.

Eine weitere besonders geeignete Klasse von Epoxidationskatalysatoren enthalten Molybdän oder Wolfram. Beispiele sind MoO₃ und WO₃, Molybdän- und Wolframsäuren, Alkali- und Erdalkalimolybdate und -wolframate, soweit ihre Basizität nicht zu einer Hydrolyse des Epoxids führt, Homo- und Heteropolymolybdate und -wolframate (= Homo- und Heteropolysäuren) und H202-Addukte der genannten Stoffklassen, wie Peroxomolybdänsäure, Peroxowolframsäure, Peroxomolybdate und Peroxowolframate, die auch in situ während der Epoxidierung aus anderen Mo- und W-Verbindungen gebildet werden können.

Der Katalysator ist üblicherweise teilchenförmig, die wirksame Komponente kann aber an den Wänden eines monolithischen Trägers mit durchgehenden Röhren oder Spalten fixiert sein. Besonders zweckmäßig werden teilchenförmige Katalysatoren in Granulat-, Kugel- oder Stäbchenform verwendet. Teilchenförmige Katalysatoren können in Form einer Festbettschüttung oder in Form eines Wirbelbetts zur Anwendung gelangen.

Das ein Olefin, Wasserstoffperoxid, Wasser und gegebenenfalls ein weiteres Gas zur Inertisierung enthaltene Gasgemisch wird dabei durch die Katalysatorschüttung geleitet oder als Wirbelschichtgas verwendet. Das Verfahren kann batchweise oder kontinuierlich durchgeführt werden. Wesentlich ist, dass sich während der Epoxidation im Reaktor, das heißt am Katalysator, keine flüssige Phase ausbildet. Hierdurch wird die Katalysatorstandzeit erhöht und der Aufwand für eine Regenerierung reduziert.

Bei Bedarf, insbesondere zur Einstellung isothermer Reaktionsbedingungen und damit Vermeidung/Verringerung der Bildung hochsiedender Nebenprodukte kann es zweckmäßig sein, das Katalysatormaterial gleichmäßig oder schichtförmig mit einem inerten oder wenig aktiven Material zu verdünnen. Beispiele geeigneter Verdünnungsmittel sind Glaspulver oder Glaskugeln, Aluminiumoxid, Siliciumdioxid und teilchenförmige mineralische oder/und silikatische Stoffe. Durch isotherme Reaktionsführung werden hot spots vermieden, was sich günstig auf die Lebensdauer des Katalysators auswirkt.

Außer durch Mitverwendung eines im wesentlichen inerten Festbettmaterials kann es vorteilhaft sein, dem das zu epoxidierende Olefin enthaltenden Gas ein Inertgas zuzumischen. Geeignete Inertgase sind beispielsweise Stickstoff, Edelgase und niedere Alkane. Die Einsatzmenge Inertgas richtet sich nach der gewünschten Reaktionstemperatur. Zur Steuerung der Katalysatoraktivität und/oder zur Modifizierung der Reaktionsbedingungen können alternativ oder zusätzlich zum Einsatz von Inertgasen auch andere Gase, wie niedrig siedende organische Lösungsmittel oder Ammoniak zugesetzt werden.

Für die Epoxidation wird gasförmiges Wasserstoffperoxid verwendet, das zweckmäßigerweise aus einer wässrigen Wasserstoffperoxidlösung, insbesondere einer solchen mit 30 bis 90 Gew.-% Wasserstoffperoxid, durch eine Verdampfung in einem dafür geeigneten Apparat gewonnen wird. Zur Verminderung von Folgereaktionen des gebildeten Epoxids mit dem aus der Verdampfung von wässrigem Wasserstoffperoxid stammendem und bei der Epoxidation aus H₂O₂ gebildeten Wasser werden bevorzugt hochkonzentrierte H₂O₂-Lösungen dem Verdampfer zugeführt. Hierdurch wird auch der Energieaufwand gemindert. Hauptfolgeprodukt ist 1,2-Propandiol. Der H₂O₂-Gehalt im Gasgemisch wird durch den Dampfdruck bei der Reaktionstemperatur begrenzt und beträgt bevorzugt 0,01 bis 25 Vol.-%, bevorzugt 0,1 bis 15 Vol-%.

Das zu epoxidierende Olefin kann in jedem Verhältnis zum Wasserstoffperoxid eingesetzt werden. Bevorzugt wird eine mindestens äquimolare Menge, besonders bevorzugt ein Olefinüberschuß. Vorzugsweise liegt das Einsatzmolverhältnis Olefin zu H₂O₂ im Bereich von größer 1 zu 1 bis 5 zu 1, insbesondere größer 1 zu 1 bis 2 zu 1.

Das zu epoxidierende Olefin enthält 2 bis 6 C-Atome, es handelt sich also um Ethen, Propen, 1-Buten, 2-Buten, Isobuten sowie Pentene und Hexene einschließlich Cyclohexen und Cyclopenten. Besonders bevorzugt eignet sich das Verfahren zur Herstellung von Propenoxid aus Propen.

Die Epoxidierung wird bei einer Temperatur unter 250 °C, vorzugsweise bei einer Temperatur im Bereich von 20 bis 200 °C und besonders bevorzugt im Bereich von 60 bis 150 °C durchgeführt. Zweckmäßigerweise erfolgt die Epoxidierung in einem Druckbereich von 1 kPa bis 2 MPa, vorzugsweise 10 kPa bis 1 MPa und besonders bevorzugt 20 kPa bis 500 kPa.

Die Aufarbeitung des Reaktionsgemischs erfolgt in dem Fachmann bekannter Weise, beispielsweise durch Kondensation des Olefinoxids und Abtrennung desselben von den übrigen Gasen oder durch einen Gaswäscher. Nicht umgesetztes Olefin kann rezykliert werden.

Zur Aufarbeitung des Reaktionsgemischs, welche eine Trennung des Olefinoxids vom nicht umgesetzten Olefin umfasst, sowie von durch eine Teilkondensation erhaltenen Gasgemischen und Kondensaten eignen sich auch bekannte Membrantrennverfahren, wie Gaspermeations-, Dampfpermeations- und Pervaporationsverfahren. Als Membran kommen solche auf der Basis von Polymeren und Molekularsieben in Betracht.

Das erfindungsgemäße Verfahren zeichnet sich durch die einfache Reaktionsführung und niedrige Reaktionstemperatur aus. Es lassen sich unterschiedliche Katalysatoren einsetzen und damit die Umsetzung auch diesbezüglich optimieren. Aufgrund der milden Reaktionsbedingungen weisen die Katalysatoren eine hohe Standzeit auf.

### Beispiele

Alle Versuche wurden in einer Glasapparatur bestehend aus einem Verdampfer, einem von außen beheizten Rohrreaktor, einer gekühlten Gaswäsche (Essigsäureethylester oder MTBE als Lösemittel) und einem Tieftemperaturkondensator durchgeführt. Es wurden handelsübliche stabilisierte Wasserstoffperoxidlösungen und Titansilikat TS-1 verwendet. Die Messung und Dosierung der Gasströme (Propen, Stickstoff) und der Wasserstoffperoxidlösung erfolgte mit Massendurchflußsensoren der Firma Bronkhorst.

### Beispiel 1

In den Verdampfer (100 °C) wurden 5 g/h einer 50 gew.-%igen Wasserstoffperoxidlösung und ein auf die Verdampfertemperatur vorgeheiztes Gasgemisch aus 2,8 Nl/h Propen und 20 Nl/h Stickstoff dosiert. Das aus dem Verdampfer austretende Gasgemisch wurde im Reaktor über eine Katalysatorschüttung aus 10 g TS-1 und 10 g Glaskugeln geleitet. Das Einsatzmolverhältnis betrug 1,7 Mol Propen pro Mol Wasserstoffperoxid. Es wurde ein Wasserstoffperoxidumsatz von 100 %, ein Propenumsatz von 25 % und eine Propenoxidausbeute, bezogen auf umgesetztes Propen, von 30 % erzielt. Als Nebenprodukte konnten Propandiol, Hydroxyaceton, Propanal, Propionsäure, Essigsäure, Ameisensäure und Formaldehyd identifiziert werden.

### Beispiel 2

In den Verdampfer (100 °C) wurden 5 g/h einer 50 gew.-%igen Wasserstoffperoxidlösung und ein auf die Verdampfertemperatur vorgeheiztes Gasgemisch aus 1,7 Nl/h Propen und 21,1 Nl/h Stickstoff dosiert. Das aus dem Verdampfer austretende Gasgemisch wurde im Reaktor über eine Katalysatorschüttung aus 10 g TS-1 und 10 g Glaskugeln geleitet. Molverhältnis Propen : H₂O₂ = 1 : 1. Der Wasserstoffperoxidumsatz betrug 100 %, der Propenumsatz 30 % und die Propenoxidausbeute, bezogen auf umgesetztes Propen, 23 %. Als Nebenprodukte konnten Propandiol, Hydroxyaceton, Propanal, Propionsäure, Essigsäure, Ameisensäure und Formaldehyd identifiziert werden.

## Patentansprüche

1. Verfahren zur Herstellung eines Olefinoxids mit 2 bis 6 C-Atomen, insbesondere Propylenoxid, durch Gasphasenepoxidation des zu epoxidierenden Olefins mit Wasserstoffperoxid, umfassend Kontaktieren eines das Olefin, Wasserstoffperoxid und Wasser enthaltenden gasförmigen Gemischs mit einem festen Katalysator und Isolieren des Olefinoxids aus dem Reaktionsgemisch,
**dadurch gekennzeichnet,**
**dass** man als Katalysator eine Verbindung eines Elements der 4. bis 6. Nebengruppe des Periodensystems der Elemente oder von Arsen oder Selen oder ein Molekularsieb verwendet und die Epoxidation bei einer Temperatur unterhalb 250 °C in Abwesenheit einer flüssigen Phase durchführt.

2. Verfahren nach Anspruch 1,
**dadurch gekennzeichnet,**
**dass** man als Katalysator einen titanhaltigen Zeolith, insbesondere Titansilikalit-1 (TS-1) mit einem TiO₂-Gehalt im Bereich von 2 bis 4 % verwendet.

3. Verfahren nach Anspruch 1,
**dadurch gekennzeichnet,**
**dass** man als Katalysator eine oxidische Verbindung von Vanadium, Niob, Tantal oder eine Molybdän- oder Wolframverbindung aus der Reihe der Oxide, Säuren, Molybdate, Wolframate, molybdän- oder wolframhaltigen Homooder Heteropolysäuren und H₂O₂-Addukten dieser Klassen verwendet.

4. Verfahren nach einem der Ansprüche 1 bis 3,
**dadurch gekennzeichnet,**
**dass** man das Gasgemisch durch ein Festbett aus teilchenförmigem Katalysator leitet.

5. Verfahren nach Anspruch 4,
**dadurch gekennzeichnet,**
**dass** das Festbett zusätzlich im wesentlichen inerte teilchenförmige Feststoffe enthält.

6. Verfahren nach einem der Ansprüche 1 bis 5,
**dadurch gekennzeichnet,**
**dass** man das Gasgemisch bei einer Temperatur im Bereich von 60 bis 150 °C mit dem Katalysator kontaktiert.

7. Verfahren nach einem der Ansprüche 1 bis 6,
**dadurch gekennzeichnet,**
**dass** man ein Gasgemisch aus dem zu epoxidierenden Olefin, Wasserstoffperoxid, Wasser und mindestens einem Inertgas aus der Reihe Stickstoff, C₁- bis C₄-Alkanen und Edelgase mit dem Katalysator kontaktiert.

8. Verfahren nach einem der Ansprüche 1 bis 7,
**dadurch gekennzeichnet,**
**dass** man das mit dem Katalysator zu kontaktierende Gasgemisch durch Verdampfen einer wässrigen Wasserstoffperoxidlösung und Zuspeisen des Olefins und bei Bedarf eines Inertgases erzeugt.

9. Verfahren nach einem der Ansprüche 1 bis 8,
**dadurch gekennzeichnet,**
**dass** das mit dem Katalysator zu kontaktierende Gasgemisch das Olefin und Wasserstoffperoxid im Molverhältnis im Bereich von größer 1 zu 1 bis 5 zu 1 enthält.

10. Verfahren nach einem der Ansprüche 1 bis 9,
**dadurch gekennzeichnet,**
**dass** man das Gasgemisch bei einem Druck im Bereich von 10 kPa bis 1 MPa mit dem Katalysator kontaktiert.

11. Verfahren nach einem der Ansprüche 1 bis 10,
**dadurch gekennzeichnet,**
**dass** man das Reaktionsgemisch oder nach einer Teilkondensation desselben erhaltene Gasgemische unter Einsatz eines Membrantrennverfahrens aufarbeitet.

## Claims

1. Process for the production of an olefinic oxide with 2 to 6 C atoms, in particular propylene oxide, by gas phase epoxidation with hydrogen peroxide of the olefin to be epoxidised, comprising contacting a gaseous mixture containing the olefin, hydrogen peroxide and water with a solid catalyst and isolating the olefinic oxide from the reaction mixture, **characterised in that** a compound of an element of subgroup IV to VI of the Periodic System of the Elements or of arsenic or selenium or a molecular sieve is used as catalyst and the epoxidation is carried out at a temperature below 250°C in the absence of a liquid phase.

2. Process according to claim 1, **characterised in that** a titanium-containing zeolite, in particular titanium silicalite-1 (TS-1) with a TiO₂ content in the range from 2 to 4% is used as catalyst.

3. Process according to claim 1, **characterised in that** an oxidic compound of vanadium, niobium, tantalum or a molybdenum or tungsten compound from the series comprising oxides, acids, molybdates, tungstates, molybdenum-containing or tungsten-containing homopolyacids or hetero-polyacids and H₂O₂ adducts of these classes is used as catalyst.

4. Process according to one of claims 1 to 3, **characterised in that** the gaseous mixture is passed through a fixed bed of particulate catalyst.

5. Process according to claim 4, **characterised in that** the fixed bed additionally contains substantially inert particulate solids.

6. Process according to one of claims 1 to 5, **characterised in that** the gaseous mixture is contacted with the catalyst at a temperature in the range from 60 to 150°C.

7. Process according to one of claims 1 to 6, **characterised in that** a gaseous phase consisting of the olefin to be epoxidised, hydrogen peroxide, water and at least one inert gas from the group comprising nitrogen, C₁-C₄ alkanes and noble gases is contacted with the catalyst.

8. Process according to one of claims 1 to 7, **characterised in that** the gaseous mixture to be contacted with the catalyst is produced by evaporating an aqueous hydrogen peroxide solution and feeding in the olefin and if necessary an inert gas.

9. Process according to one of claims 1 to 8, **characterised in that** the gaseous mixture to be contacted with the catalyst contains the olefin and hydrogen peroxide in a molar ratio in the range of greater than 1:1 to 5:1.

10. Process according to one of claims 1 to 9, **characterised in that** the gaseous mixture is contacted with the catalyst at a pressure in the range from 10 kPa to 1 MPa.

11. Process according to one of claims 1 to 10, **characterised in that** the reaction mixture or gaseous mixtures obtained after a partial condensation of the latter is/are worked up by using a membrane separation process.

## Revendications

1. Procédé de fabrication d'un oxyde d'oléfïne comprenant 2 à 6 atomes de C, notamment de l'oxyde de propylène, à l'aide d'une époxydation en phase gazeuse de l'oléfine à époxyder avec du peroxyde d'hydrogène, comprenant la mise en contact d'un mélange gazeux contenant l'oléfine, du peroxyde d'hydrogène et de l'eau avec un catalyseur solide et une isolation de l'oxyde d'oléfine du mélange réactif,
**caractérisé en ce que**
l'on utilise en tant que catalyseur un composé constitué d'un des éléments du 4^{e} au 6^{e} co-groupe de la classification périodique des éléments ou de l'arsenic ou de sélénium ou encore un tamis moléculaire, et on effectue l'époxydation à une température inférieure à 250°C en l'absence d'une phase liquide.

2. Procédé selon la revendication 1,
**caractérisé en ce que**
comme catalyseur on utilise une zéolithe titanifère, notamment du silicalite-1 de titane (TS-1) avec une teneur en TiO₂ dans une plage de 2 à 4 %.

3. Procédé selon la revendication 1,
**caractérisé en ce que**
comme catalyseur on utilise une combinaison par voie d'oxydation de vanadium, de niobium, de tantale ou un composé de molybdène ou de tungstène du groupe des oxydes, des acides, des molybdates, des tungstates, des acides homopolaires ou hétéropolaires contenant du molybdène ou du tungstène et des adductions de H₂O₂ de ces catégories.

4. Procédé selon l'une quelconque des revendications 1 à 3,
**caractérisé en ce que**
le mélange gazeux est conduit à travers un lit solide constitué d'un catalyseur en forme de particules.

5. Procédé selon la revendication 4,
**caractérisé en ce que**
le lit solide contient en complément des substances solides en forme de particules essentiellement inertes.

6. Procédé selon l'une quelconque des revendications 1 à 5,
**caractérisé en ce qu'**
on met en contact le mélange gazeux avec un catalyseur à une température dans une plage de 60 à 150°C.

7. Procédé selon l'une quelconque des revendications 1 à 6,
**caractérisé en ce qu'**
on met en contact avec le catalyseur un mélange gazeux constitué de l'oléfine à époxyder, du peroxyde d'hydrogène, de l'eau et au moins d'un gaz inerte du groupe, azote, alcanes en C₁ à C₄ et gaz nobles.

8. Procédé selon l'une quelconque des revendications 1 à 7,
**caractérisé en ce que**
le mélange gazeux à mettre en contact avec le catalyseur est produit par l'évaporation d'une solution aqueuse de peroxyde d'hydrogène et moyennant l'adduction de l'oléfine et, en cas de besoin, d'un gaz inerte.

9. Procédé selon l'une quelconque des revendications 1 à 8,
**caractérisé en ce que**
le mélange gazeux à mettre en contact avec le catalyseur contient l'oléfine et le peroxyde d'hydrogène dans un rapport molaire dans une plage supérieur à 1 : 1 à 5 : 1.

10. Procédé selon l'une quelconque des revendications 1 à 9,
**caractérisé en ce qu'**
on met le mélange gazeux en contact avec le catalyseur à une pression dans une plage de 10 kPA à 1 Mpa.

11. Procédé selon l'une quelconque des revendications 1 à 10,
**caractérisé en ce que**
le mélange réactif ou des mélanges gazeux obtenus après une condensation partielle de celui-ci, sont traités en employant un procédé de séparation par membrane.
